# EUROPEAN PATENT SPECIFICATION

(11) **EP 4 053 466 B1**
(45) Date of publication and mention of the grant of the patent: **23.07.2025**
(21) Application number: 21159866.9
(22) Date of filing: 01.03.2021
(51) Int. Cl.: F24F 8/22, F24F 13/06, F24F 13/078, F24F 13/20, F24F 13/28, F24F 3/167

(54) **AIR TERMINAL DEVICE WITH INTEGRAL LIGHT FIXTURE**
LUFTAUSLASSVORRICHTUNG MIT INTEGRIERTER LEUCHTE
DISPOSITIF DE TERMINAL D'AIR À LUMINAIRE INTÉGRÉ

(43) Date of publication of application: 07.09.2022
(73) Proprietor: Halton OY, 47400 Kausala (FI)
(72) Inventor: Hagström, Kim, 47400 Kausala (FI)
(74) Representative: Papula Oy

(56) References cited:
- EP-A1- 3 093 574
- WO-A1-98/38458
- CN-A- 107 726 130
- CN-U- 203 963 886
- US-A1- 2017 321 877
- US-A1- 2019 168 160
- US-A1- 2020 179 544

## Description

### TECHNICAL FIELD

The present invention relates to an air terminal device with integral light fixture for a clean room.

### BACKGROUND OF THE ART

A clean room, such as a room for research or production or room in a hospital, such as operating room or patient room, has high hygiene demands. Such rooms have need for both air purification and surface cleaning. In traditional approach air purification and surface cleaning by irradiation (UV, Blue light) as well as room lighting are used with separate systems with individual installation. In above mentioned cleanrooms constructional tightness - especially ceiling tightness and product maintainability are essential requirements to ensure clean operations. Having separate systems add significant complexity and challenge to ceiling design and integration. Firstly, a hole in the ceiling is a source for leakage and impairs the loadbearing capability of the ceiling. Secondly, the air supply and exhaust arrangements are the top priority in the rooms with highest cleanliness, and any other ceiling installation add challenge to room airflow control.

Typically, the disinfection and lighting arrangements are used continuously at a high intensity. The light fixtures are installed in a separate enclosure next to an air device and they are continuously at a high temperature, which pose a risk for shortened lifetime for light fixtures. Also, in such an arrangement light fixture maintenance would require access to ceiling void by opening the ceiling construction.

Document US 2017/0321877 A1 discloses one example of an air recirculating device having light sources. Document CN 203 963 886 U discloses an air terminal device according to the preamble of claim 1.

### OBJECTIVE OF THE INVENTION

The objective of the device is to alleviate the disadvantages mentioned above.

In particular, it is an objective of the present device to provide an air terminal device with efficient air diffusion and surface disinfection capabilities.

### SUMMARY

The invention is defined in claim 1. In particular, according to a first aspect, the present invention provides an air terminal device with integral light fixture for a clean room, comprising a housing having a cover panel comprising at least one opening, a light fixture for providing light outside of the air terminal device, an air chamber configured to receive air and to discharge air out of the air chamber. The air is configured to be transferred through the at least one opening in the cover panel. The light fixture is arranged inside the air chamber, and the cover panel comprises at least one light fixture opening, in which the light fixture is integrated.

The advantage of the invention is that air diffusion and surface disinfection may be provided efficiently with one integrated device.

According to the invention, the light fixture is arranged within the light fixture opening so that the perimeter of the light fixture is at the same level as the cover panel or projecting from the cover panel. The advantage is that the light may be distributed on wider area and even along the surface of the cover panel. Thus, the light may be provided as with using separate lighting.

According to the invention, the cover panel is detachably connected to the housing whereby the air chamber and the light fixture is exposed when the cover panel is removed. The advantage is that the air chamber and the light fixture are accessible without need to disassemble the device or access to the contaminated ceiling void.

In an embodiment of the device, the cover panel is hingedly connected to the housing.

In an embodiment of the device, the light fixture is detachable. The advantage is that the light fixture may be replaced and the space behind the light fixture is accessible without need to access the contaminated ceiling void.

In an embodiment of the device, the light fixture is hingedly connected to the air terminal device.

In an embodiment of the device, the light fixture is configured to be flushed with the received air before the air is diffused out of the air terminal device. The advantage is that the lifetime of the light fixture may be extended when the heat from the light fixture is removed by the air flow. Especially when cooled supply air is transferred through the air terminal device, the cooled supply air removed heat from the light fixture efficiently.

In an embodiment of the device, the cover panel comprises plurality of openings configured to provide adjustable air flow pattern. Advantage is that the air flow pattern may be adjusted to meet the requirements of the clean room.

In an embodiment of the device, the device comprises a filter configured to filter the air received into the air chamber. The advantage is that the impurities may be removed from the air before it is discharged into the clean room.

In an embodiment of the device, the filter is inside the air chamber. The advantage is that the filter is more easily accessible.

In an embodiment of the device, the light fixture is between the filter and the cover panel. The advantage is that the air is filtered before the light fixture is flushed with the air flow and, therefore, the light fixture remains cleaner and the impurities are not collected on the surface of the light fixture, which would increase the temperature of the light fixture even further.

In an embodiment of the device, the light fixture is configured to provide light to the filter. The advantage is that the filter may be disinfected by the same light fixture and, thus, the filter remains cleaner and/or the filter needs to be replaced less frequently.

In an embodiment of the device, the light fixture is configured to produce visible light.

In an embodiment of the device, the light fixture is configured to produce disinfection light.

In an embodiment of the device, the light fixture is configured to produce visible light and disinfection light.

In an embodiment of the device, the device comprises a control unit configured to adjust the light fixture to produce visible light or disinfection light.

In an embodiment of the device, the air chamber is a supply air chamber configured to receive supply air and to discharge the supply air out of the device through the at least one opening of the cover panel.

It is to be understood that the aspects and embodiments of the invention described above may be used in any combination with each other as long as within the scope of the claims. Several of the aspects and embodiments may be combined together to form a further embodiment of the invention as long as within the scope of the claims.

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
**Fig. 1** shows an air terminal device according to the invention,
**Fig. 2** shows the air terminal device wherein the cover panel is removed completely,
**Fig. 3** shows the air terminal device wherein the cover panel is removed, and the light fixture is pivoted, with a hinge,
**Fig. 4** is a cross-sectional view of the air terminal device from one end.
**Fig. 5** is a cross-sectional view of the air terminal device from one end, wherein the device comprises a filter,
**Fig. 6** shows an embodiment of the air terminal device, wherein the device comprises two cover panels and two light fixtures, and
**Fig. 7** shows an embodiment of the air terminal device, wherein the device comprises several cover panels and several light fixtures, and the device is configured in rectangular form.

### DETAILED DESCRIPTION

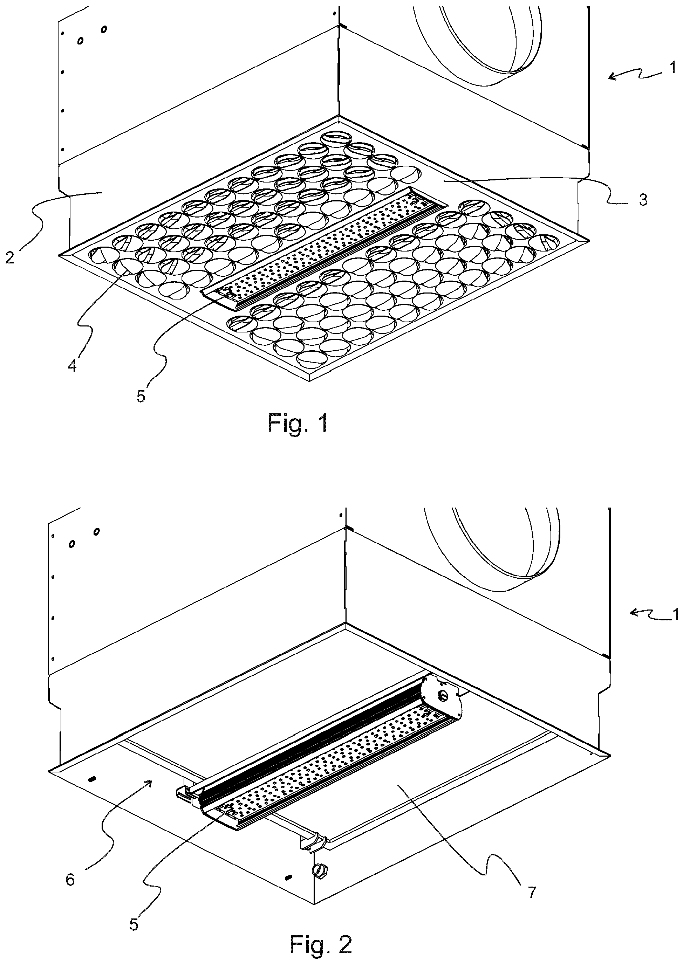

Figure 1 shows an air terminal device 1 with integral light fixture 5 for a clean room. The air terminal device may be a supply air device for providing supply air into the clean room or it may be exhaust air device for removing room exhaust air from the clean room. In both applications, the basic structure of the device is similar, and the air flow direction is the only one that changes. For air supply, the air is received from, for example, a supply air duct and discharged into the clean room, and for air exhaust, the exhaust air is received from the clean room and discharged into, for example, an exhaust duct.

The air terminal device comprises a housing 2 which forms the outer casing of the device. The housing 2 comprises a cover panel 3, which is exposed to the clean room when the device is installed in the clean room. The cover panel 3 comprises at least one opening 4, through which air is transferred into the air terminal device or out of the air terminal device. The air transferred through the at least one opening 4 may be exhaust room air from the clean room or the air may be supply air from supply air duct of the building. The cover panel 3 may comprise plurality of openings 4, as seen in figure 1. The air terminal device comprises an air chamber 6 which is arranged to receive air and to discharge the air out of the air chamber 6. The air chamber 6 is covered by the cover panel 3 and the cover panel 3 is detachably connected to the housing 2, i.e. the cover panel 3 is openable. The cover panel 3 may be connected to the housing 2 so that it may be completely removed from the air terminal device or it may be connected to the housing 2 by hinge or hinges so that it may be removed by pivoting it from one edge while one edge remains connected to the housing 2 by the hinge or hinges. In any case, removing (completely or hingedly) the cover panel 3 exposes the air chamber 6 of the air terminal device.

The cover panel 3 may be perforated panel having plurality of perforations, through which the supply air may be discharged into the room or room the exhaust air from the room may be received into the air terminal device.

In case that the air terminal device is for providing a supply air into the clean room, the openings 4 in the cover panel 3 may be used for providing desired air flow pattern from the device. The openings 4 of the cover panel 3 may comprise rotatable nozzles for directing the air jets. The clean rooms are used for highly critical operations and, therefore, it is beneficial to have possibility to adjust the air flow pattern so that air flows are directed in desired directions.

The air terminal device may comprise a plenum 10 which is configured to provide supply air to the air chamber 6 or to receive room exhaust air from the air chamber 6. Optionally, the plenum 10 may be configured to provide air to several air chambers 6 in case that the air terminal device comprises one plenum 10 and several air chambers 6, which embodiments will be discussed later.

The air terminal device comprises a light fixture 5 for providing light outside of the air terminal device, i.e. into the clean room, and the light fixture 5 is arranged inside the air chamber 6 and integrated with a light fixture 5 opening. This should be understood so that the light fixture 5 is mainly inside the air chamber 6 and some parts, e.g. light diffuser or cover, are at the same level as the cover panel or even projecting from the cover panel 3, if such components are part of the light fixture 5. The main parts, e.g. light source and electricity are inside the air chamber 6. The cover panel 3 comprises at least one light fixture opening in which the light fixture 5 is integrated, and the light is provided outside of the air terminal device i.e. into the clean room. The light fixture 5 may be integrated to the cover panel 3 so that the light fixture 5 may be removed from the device by removing the cover panel 3. Optionally, the light fixture 5 is connected to the housing and by removing the cover panel 3, the light fixture 5 inside the air chamber 6 is completely exposed and the light fixture 5 may be removed from the air terminal device or it may be inspected or the lightning source, e.g. lamp, may be replaced. By placing the light fixture 5 inside the air chamber 6, the air flow inside the air chamber 6 may be used for removing heat, i.e. cooling, from the light fixture 5, i.e. the light fixture 5 is flushed by the air, and, thus, preventing overheating of the light fixture 5. As the light fixture 5 may be kept at lower temperature, the lifetime of the light fixture 5 may be extended. When the air terminal device is a supply air device, the supply air may be cooled air, which is transferred through the device. The cooled supply air removes heat efficiently from the light fixture when the light fixture is flushed with the cooled supply air.

The light fixture 5 comprises a light source or light sources, which may comprise a light-emitting diode (LED) unit having a number of LEDs. The light fixture 5 may be configured to provide visible light (wavelengths in range of 400-700 nm) or disinfection light, such as blue light (wavelengths in range of 400-490 nm) or UV light (wavelengths in range of 220-300 nm), which have been shown to destroy impurities, such as bacteria. Optionally, the light fixture 5 may be configured to provide visible light and disinfection light. The light fixture 5 may be configured to provide disinfection light at least on two different wavelengths.

The air terminal device 1 may comprise a control unit, which may be configured adjust the operation of the air terminal device 1. The control unit may be configured to adjust the light fixture 5 to produce visible light or disinfection light based on the schedule of the clean room. For example, the visible light and the disinfection light may be configured to be produced based on schedule of operations, i.e. disinfection light is produced when disinfection is needed and visible light when only visible light is needed. The control unit may also be configured to adjust and/or schedule the air ventilation together with the light fixture, i.e. supplying the supply air into the room or exhaust room air into the air terminal device.

Figure 2 shows the air terminal device 1 wherein the cover panel 3 is removed completely, and the air chamber 6 and the light fixture 5 is exposed. The light fixture 5 may be detachable so that it may be removed completely and/or replaced if needed. The light fixture 5 is connected to the frame, i.e. to opposite side walls of the air chamber 6, e.g. by hinge 8 or hinges so that it may be pivoted from one end while the other end remains connected to the frame by the hinge 8 or hinges.

The air terminal device 1 may comprise a filter 7, which is configured to filter the air received into the air chamber 6 before it is discharged into the clean room. The filter 7 may be inside the air chamber 6 or it may be outside of the air chamber 6 so that the air is filtered before it is received into the air chamber 6. By filtering the air, impurities, such as dust, particles, bacteria, viruses etc. may be removed from the air and the discharged air meets the requirements regarding cleanliness or the supply air.

The filter 7 may be placed in parallel to the cover panel 3 so that it covers the whole cross-sectional area of the air chamber 6. Thus, the filter 7 surface area may be larger and, thus, its capability to filter impurities is better.

The filter 7 may be high-efficiency particulate air (HEPA) filter.

When the filter 7 is placed inside the air chamber, the light fixture may be configured to provide light to the filter 7 for disinfecting the filter 7. Optionally, another separate light fixture may be provided to provide disinfection light to the filter 7.

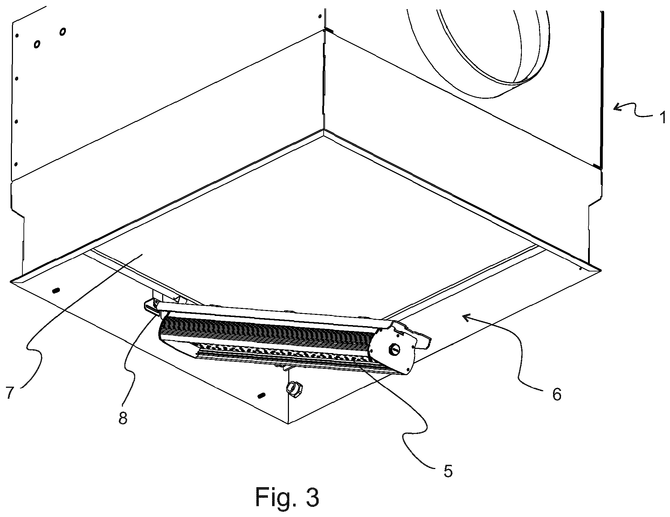

Figure 3 shows the air terminal device 1 wherein the cover panel 3 is removed, and the light fixture 5 is pivoted from one end. By removing the cover panel 3 and the light fixture 5, the filter 7 is accessible, and it may be removed, inspected, or replaced without disassembling the whole air terminal device 1 or without need to access inside the ceiling.

The light fixture 5 may be arranged between the filter 7 and the cover panel 3, whereby the impurities in the air are filtered before the light fixture 5 and the light fixture 5 remains cleaner.

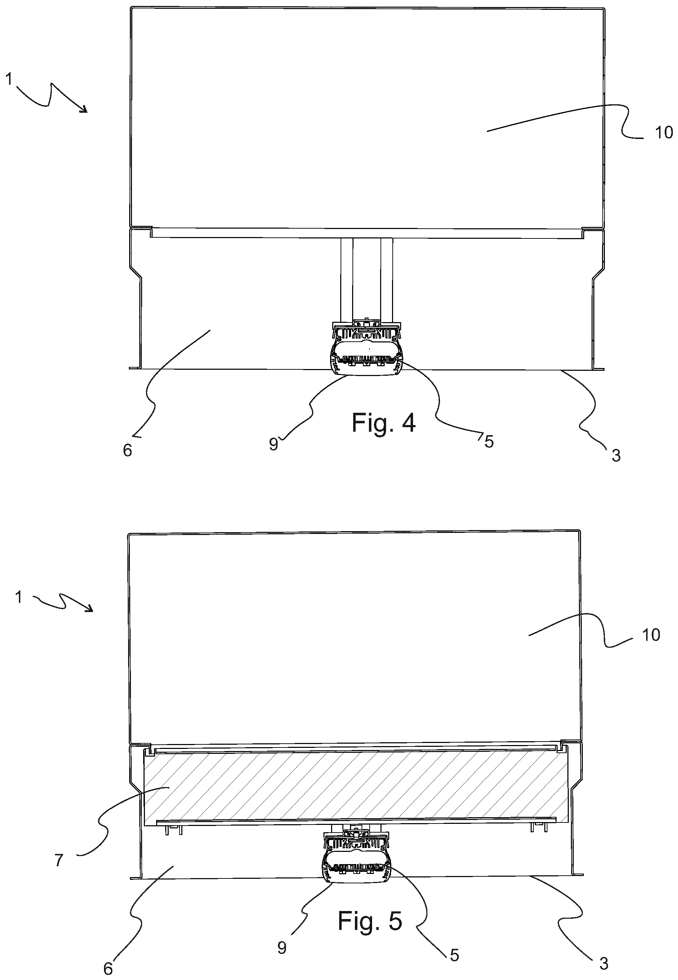

Figure 4 is a cross-sectional view of the air terminal device 1 viewed from one end. The light fixture 5 is arranged inside the air chamber 6 so that the air flow inside the air chamber 6 flushes and, thus, removes heat from the light fixture 5. The light fixture 5 is arranged so that the perimeter 9 of the light fixture 5 is at the same level as the cover panel 3, or it is arranged so that the perimeter 9 of the light fixture 5 is projecting from the cover panel 3, as seen in figure 4 If the perimeter 9 of the light fixture 5 is projecting from the cover panel 3, the light may be provided to larger area and even along the cover panel 3 surface.

Figure 5 is the same cross-sectional view of the air terminal device 1 as in figure 4. In this figure, the filter 7 is provided inside the air chamber 6 and the light fixture 5 is between the filter 7 and the cover panel 3.

The features and embodiments discussed above may be implemented in embodiments wherein the air terminal device 1 comprises two or more cover panel 3s and two or more light fixtures 5, and wherein the air terminal device 1 has different shapes, as will be discussed in the following section.

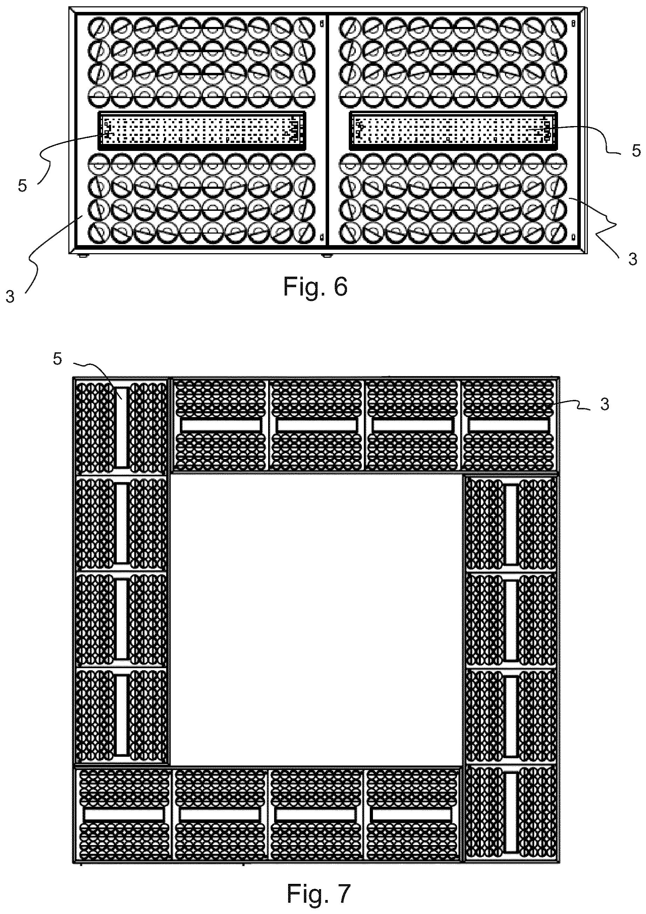

Figure 6 shows an embodiment of the air terminal device 1. In this embodiment, the air terminal device 1 comprises two cover panels 3 and two light fixtures 5 adjacent to each other. The air terminal device 1 may also comprise more than two cover panels 3 and more than two light fixtures 5. The number of separate cover panels 3 may be the same as the number of separate light fixtures 5. Optionally, each cover panel 3 comprises several light fixture openings through which the light is provided from several light fixtures 5.

The air terminal device 1 having two or more cover panels 3 and two or more light fixtures 5, may comprise only one air chamber 6 and each light fixture 5 is arranged inside this one air chamber 6. Optionally, the air terminal device 1 may comprise several air chambers 6 so that at least one light fixture 5 is arranged inside each air chambers 6.

Figure 7 shows an embodiment of the air terminal device 1. In this embodiment, the air terminal device 1 comprises several cover panels 3 and several light fixtures 5. The air terminal device 1 is configured in rectangular shape so that it may be installed to the ceiling of a clean room so that it surrounds a critical area, for example operating table in hospital, below. Optionally, the air terminal device 1 may be configured so that it has two or more separate elongated sections, as seen for example in figure 6, and they may be installed to the ceiling of a clean room so that a critical area below the air terminal device 1 is between the separate sections, e.g. two sections are on opposite sides of the critical area.

## Claims

1. An air terminal device (1) with integral light fixture for a clean room, wherein the air terminal device is a supply air device for providing supply air into the clean room, or an exhaust air device for removing room exhaust air from the clean room,
comprising
- a housing (2) having a cover panel (3) comprising at least one opening (4),
- a light fixture (5) for providing light outside of the air terminal device (1),
- an air chamber (6) configured to receive air and to discharge air out of the air chamber,
wherein the device is configured to transfer air through the at least one opening (4) in the cover panel (3),
wherein the cover panel (3) comprises at least one light fixture opening,
wherein the light fixture (5) is arranged within the at least one light fixture opening so that the perimeter (9) of the light fixture (5) is at the same level as the cover panel (3) or projecting from the cover panel (3),
**characterized in that**
- the light fixture (5) is arranged inside the air chamber (6) so that the light fixture is connected to opposite side walls of the air chamber (6),
- the cover panel (3) is detachably connected to the housing (2) whereby the air chamber (6) and the light fixture (5) are exposed when the cover panel (3) is removed.

2. The air terminal device according to claim 1, wherein the cover panel (3) is hingedly connected to the housing (2).

3. The air terminal device according to any of claims 1 to 2, wherein the light fixture (5) is detachable.

4. The air terminal device according to any of claims 1 to 3, wherein the light fixture (5) is hingedly connected to the air terminal device.

5. The air terminal device according to any of claims 1 to 4, wherein the light fixture (5) is configured to be flushed with the received air, such that the air flow inside the air chamber cools the light fixture, before the air is diffused out of the air terminal device (1).

6. The air terminal device according to any of claims 1 to 5, wherein the cover panel (3) comprises plurality of openings (4) configured to provide adjustable air flow pattern.

7. The air terminal device according to any of claims 1 to 6, comprising a filter (7) configured to filter the air received into the air chamber (6).

8. The air terminal device according to claim 7, wherein the filter (7) is inside the air chamber (6).

9. The air terminal device according to claim 7 or 8, wherein the light fixture (5) is between the filter (7) and the cover panel (3).

10. The air terminal device according to claim 8 or 9, wherein the light fixture (5) is configured to provide light to the filter (7).

11. The air terminal device according to any of claims 1 to 10, wherein the light fixture (5) is configured to produce visible light.

12. The air terminal device according to any of claims 1 to 10, wherein the light fixture (5) is configured to produce disinfection light.

13. The air terminal device according to any of claims 1 to 10, wherein the light fixture (5) is configured to produce visible light and disinfection light.

14. The air terminal device according to claim 13, comprising a control unit configured to adjust the light fixture (5) to produce said visible light or said disinfection light.

15. The air terminal device according to any of claims 1 to 14, wherein the air chamber (6) is a supply air chamber configured to receive supply air and to diffuse supply air out of the device through the at least one opening of the cover panel (3).

## Patentansprüche

1. Luftauslassvorrichtung (1) mit integrierter Leuchte für einen Reinraum, wobei die Luftauslassvorrichtung eine Zuluftvorrichtung zum Bereitstellen von Zuluft in den Reinraum, oder eine Abluftvorrichtung zum Entfernen von Raumabluft aus dem Reinraum ist, umfassend:
- ein Gehäuse (2), das ein Abdeckungspaneel (3) aufweist, das mindestens eine Öffnung (4) umfasst,
- eine Leuchte (5) zum Bereitstellen von Licht außerhalb der Luftauslassvorrichtung (1),
- eine Luftkammer (6), die dazu konfiguriert ist, Luft aufzunehmen und Luft aus der Luftkammer auszulassen,
wobei die Vorrichtung dazu konfiguriert ist, Luft durch die mindestens eine Öffnung (4) in dem Abdeckungspaneel (3) zu leiten,
wobei das Abdeckungspaneel (3) mindestens eine Leuchtenöffnung umfasst, wobei die Leuchte (5) innerhalb der mindestens einen Leuchtenöffnung angeordnet ist, derart, dass der Perimeter (9) der Leuchte (5) auf dem gleichen Niveau wie das Abdeckungspaneel (3) ist oder von dem Abdeckungspaneel (3) hervorsteht,
**dadurch gekennzeichnet, dass**
- die Leuchte (5) innerhalb der Luftkammer (6) angeordnet ist, derart, dass die Leuchte mit gegenüberliegenden Seitenwänden der Luftkammer (6) verbunden ist,
- das Abdeckungspaneel (3) abnehmbar mit dem Gehäuse (2) verbunden ist, wobei die Luftkammer (6) und die Leuchte (5) freigelegt sind, wenn das Abdeckungspaneel (3) entfernt ist.

2. Luftauslassvorrichtung gemäß Anspruch 1, wobei das Abdeckungspaneel (3) gelenkig mit dem Gehäuse (2) verbunden ist.

3. Luftauslassvorrichtung gemäß einem der Ansprüche 1 bis 2, wobei die Leuchte (5) abnehmbar ist.

4. Luftauslassvorrichtung gemäß einem der Ansprüche 1 bis 3, wobei die Leuchte (5) gelenkig mit der Luftauslassvorrichtung verbunden ist.

5. Luftauslassvorrichtung gemäß einem der Ansprüche 1 bis 4, wobei die Leuchte (5) dazu konfiguriert ist, mit der aufgenommenen Luft umspült zu werden, derart, dass der Luftstrom innerhalb der Luftkammer die Leuchte kühlt, bevor die Luft aus der Luftauslassvorrichtung (1) diffundiert wird.

6. Luftauslassvorrichtung gemäß einem der Ansprüche 1 bis 5, wobei das Abdeckungspaneel (3) eine Mehrzahl von Öffnungen (4) umfasst, die dazu konfiguriert ist, anpassbare Luftstrommuster bereitzustellen.

7. Luftauslassvorrichtung gemäß einem der Ansprüche 1 bis 6, umfassend einen Filter (7), der dazu konfiguriert ist, die in die Luftkammer (6) aufgenommene Luft zu filtern.

8. Luftauslassvorrichtung gemäß Anspruch 7, wobei der Filter (7) sich innerhalb der Luftkammer (6) befindet.

9. Luftauslassvorrichtung gemäß Anspruch 7 oder 8, wobei sich die Leuchte (5) zwischen dem Filter (7) und dem Abdeckungspaneel (3) befindet.

10. Luftauslassvorrichtung gemäß Anspruch 8 oder 9, wobei die Leuchte (5) dazu konfiguriert ist, Licht für den Filter (7) bereitzustellen.

11. Luftauslassvorrichtung gemäß einem der Ansprüche 1 bis 10, wobei die Leuchte (5) dazu konfiguriert ist, sichtbares Licht zu produzieren.

12. Luftauslassvorrichtung gemäß einem der Ansprüche 1 bis 10, wobei die Leuchte (5) dazu konfiguriert ist, Desinfektionslicht zu produzieren.

13. Luftauslassvorrichtung gemäß einem der Ansprüche 1 bis 10, wobei die Leuchte (5) dazu konfiguriert ist, sichtbares Licht und Desinfektionslicht zu produzieren.

14. Luftauslassvorrichtung gemäß Anspruch 13, umfassend einer Steuereinheit, die dazu konfiguriert ist, die Leuchte (5) dazu anzupassen, das sichtbare Licht oder das Desinfektionslicht zu produzieren.

15. Luftauslassvorrichtung gemäß einem der Ansprüche 1 bis 14, wobei die Luftkammer (6) eine Zuluftkammer ist, die dazu konfiguriert ist, Zuluft aufzunehmen und Zuluft aus der Vorrichtung durch die mindestens eine Öffnung des Abdeckungspaneels (3) zu diffundieren.

## Revendications

1. Dispositif de terminal d'air (1) à luminaire intégré pour une salle blanche, dans lequel le dispositif de terminal d'air est un dispositif de distribution d'air pour fournir de l'air de distribution dans la salle blanche, ou un dispositif d'extraction d'air pour éliminer l'air d'extraction de salle de la salle blanche, comprenant
- un boîtier (2) présentant un panneau de couverture (3) comprenant au moins une ouverture (4),
- un luminaire (5) pour fournir de la lumière à l'extérieur du dispositif de terminal d'air (1),
- une chambre d'air (6) configurée pour recevoir de l'air et pour évacuer l'air hors de la chambre d'air, dans lequel le dispositif est configuré pour transférer de l'air à travers l'au moins une ouverture (4) dans le panneau de couverture (3),
dans lequel le panneau de couverture (3) comprend au moins une ouverture de luminaire, dans lequel le luminaire (5) est disposé à l'intérieur de l'au moins une ouverture de luminaire de telle sorte que le périmètre (9) du luminaire (5) est au même niveau que le panneau de couverture (3) ou fait saillie depuis le panneau de couverture (3),
**caractérisé en ce que**
- le luminaire (5) est disposé à l'intérieur de la chambre d'air (6) de telle sorte que le luminaire est relié à des parois latérales opposées de la chambre d'air (6),
- le panneau de couverture (3) est relié amovible au boîtier (2) de sorte que la chambre d'air (6) et le luminaire (5) sont mis à nu lorsque le panneau de couverture (3) est retiré.

2. Dispositif de terminal d'air selon la revendication 1, dans lequel le panneau de couverture (3) est relié articulé au boîtier (2).

3. Dispositif de terminal d'air selon l'une quelconque des revendications 1 à 2, dans lequel le luminaire (5) est amovible.

4. Dispositif de terminal d'air selon l'une quelconque des revendications 1 à 3,
dans lequel le luminaire (5) est relié articulé au dispositif de terminal d'air.

5. Dispositif de terminal d'air selon l'une quelconque des revendications 1 à 4, dans lequel le luminaire (5) est configuré pour être balayé par l'air reçu, de telle sorte que le flux d'air à l'intérieur de la chambre d'air refroidit le luminaire, avant que l'air ne soit diffusé hors du dispositif de terminal d'air (1).

6. Dispositif de terminal d'air selon l'une quelconque des revendications 1 à 5, dans lequel le panneau de couverture (3) comprend une pluralité d'ouvertures (4) configurées pour fournir un motif de flux d'air réglable.

7. Dispositif de terminal d'air selon l'une quelconque des revendications 1 à 6, comprenant un filtre (7) configuré pour filtrer l'air reçu dans la chambre d'air (6).

8. Dispositif de terminal d'air selon la revendication 7, dans lequel le filtre (7) est à l'intérieur de la chambre d'air (6).

9. Dispositif de terminal d'air selon la revendication 7 ou la revendication 8, dans lequel le luminaire (5) est entre le filtre (7) et le panneau de couverture (3).

10. Dispositif de terminal d'air selon la revendication 8 ou la revendication 9, dans lequel le luminaire (5) est configuré pour fournir de la lumière au filtre (7).

11. Dispositif de terminal d'air selon l'une quelconque des revendications 1 à 10, dans lequel le luminaire (5) est configuré pour produire une lumière visible.

12. Dispositif de terminal d'air selon l'une quelconque des revendications 1 à 10, dans lequel le luminaire (5) est configuré pour produire une lumière de désinfection.

13. Dispositif de terminal d'air selon l'une quelconque des revendications 1 à 10, dans lequel le luminaire (5) est configuré pour produire une lumière visible et une lumière de désinfection.

14. Dispositif de terminal d'air selon la revendication 13, comprenant une unité de commande configurée pour ajuster le luminaire (5) pour produire ladite lumière visible ou ladite lumière de désinfection.

15. Dispositif de terminal d'air selon l'une quelconque des revendications 1 à 14, dans lequel la chambre d'air (6) est une chambre de distribution d'air configurée pour recevoir de l'air de distribution et pour diffuser de l'air de distribution hors du dispositif à travers l'au moins une ouverture du panneau de couverture (3).
